# EUROPEAN PATENT SPECIFICATION

(11) **EP 0 780 107 B1**
(45) Date of publication and mention of the grant of the patent: **27.02.2002**
(21) Application number: 96118776.2
(22) Date of filing: 22.11.1996
(51) Int. Cl.: A61F 9/00, A61B 17/11, A61B 17/122, A61F 9/007, A61B 17/04

(54) **Muscle clamping apparatus**
Muskelklemmvorrichtung
Dispositif de serrage de muscle

(30) Priority: 24.11.1995 KR 9543460
(43) Date of publication of application: 25.06.1997
(73) Proprietor: Park, Jong-deok, Seo-gu, Taejon (KR)
(72) Inventor: Min, Byung-moo, Seo-gu, Taejon (KR)
(74) Representative: Viering, Jentschura & Partner

(56) References cited:
- EP-A- 0 130 037
- EP-A- 0 315 465
- WO-A-95/17865
- FR-A- 2 322 578
- US-A- 4 506 670
- US-A- 4 935 028

## Description

The invention relates to a muscle clamping apparatus according to the precharacterizing part of claim 1.

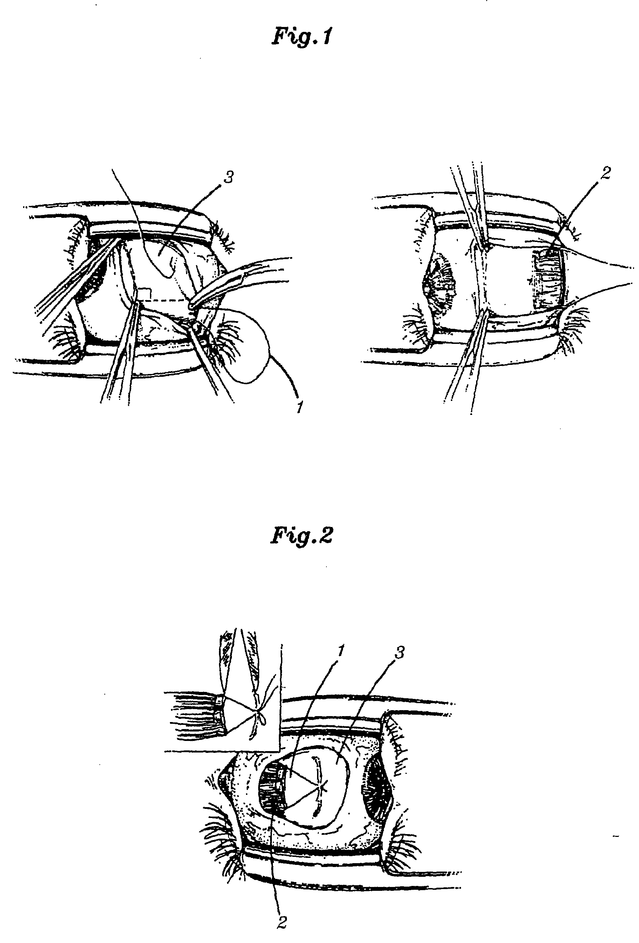

The conventional strabismus surgery requires suture and needle 1, as illustrated in Fig. 1, to be weaved in and out of the extra ocular muscle 2, while passing the needle through the sclera 3 (the tough coat of the eye) and thereby fixing the muscle 2 to the sclera 3.

This method brings about potential complications. To begin with, the suture binding the extraocular muscle 2 may get so loose that strabismus surgery may be performed incorrect; whose result may suggest the case of resurgery. Then, endophthalmitis may occur in penetrating the sclera when the needle goes through it, which is only 0.3 mm thick; and penetration may be either too deep or too shallow. And serious complications may occur such as separation of the retina, partial death necrosis of the retina, anterior hemorrhage, dislocation of the lens, and even blindness and loss of the eyeball.

Conventionally, the type of the hang-loose has been employed to solve such problems. In accordance with Fig. 2, this is accomplished by first binding the extraocular muscle 2 by suture and needle 1, but not passing the needle through the sclera 3; then hanging loose the muscular portion attached to the muscle 2; and finally cutting off this portion, which is posteriorly to be resecured further back by means of suture.

The hang-loose has such a disadvantage that it makes narrow the muscular width of the extraocular muscle 2, depending on stretching the suture 1 and therefore the movement of the ocular muscle becomes unstable after it is resecured.

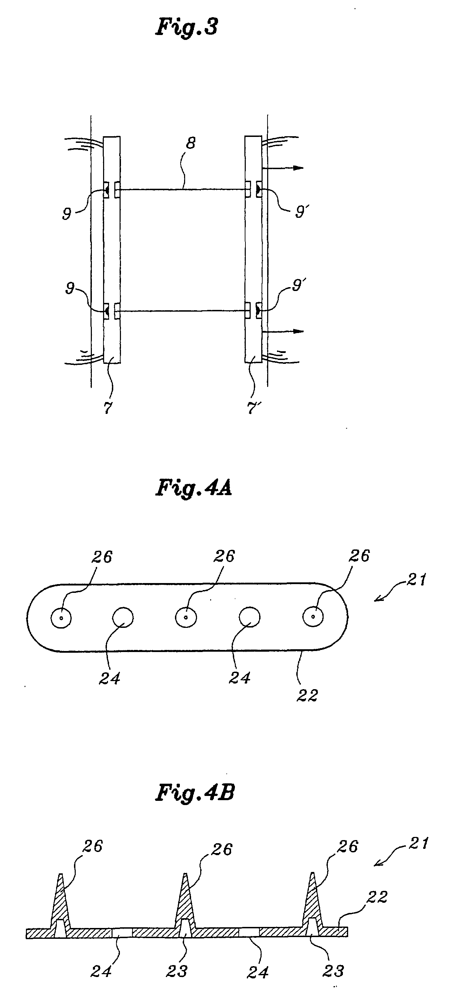

For this case, Lingua in the U.S. Patent No. 4,519,392 issued on May 28, 1985 shows a type of operation by using a clip, not binding the ocular muscle by suture, entitled "Hemostasing Muscle Clips for Needleless Surgery", as illustrated in Fig. 3. According to this type, two clips 7 and 7' are to clamp each side of the ocular muscle; then, the muscle between the two clips are cut off; and the length of the suture 8 comes to be adjusted according to its amount as needed, so that after fixing its two ends to the holes 9 and 9' of each clip, it is pulled backward.

This operation appears complicated not only in the method for two clips to clip the ocular muscle, but also in the method for manipulating two parallel sutures to thereby stretch them out to the same length as needed. So it become not utilized due to complexities and difficulties.

FR-A-2 322 578 discloses a muscle clamping apparatus comprising first and second fixing means and first and second supporting means for supporting the first and second fixing means.

An object of this invention is to provide for a muscle clamping apparatus capable of clipping the muscle effectively, for example, the ocular muscle only by simple manipulation.

According to the invention, this is achieved by the features in the characterizing part of claim 1. Advantageous further embodiments are described in the subclaims.
Figs. 1A and 1B show essential steps of a prior art strabotomy;
Fig. 2 shows an essential step of another prior art strabotomy;
Fig. 3 shows a scheme of a clip popular used in the typical prior art surgical operation;
Figs. 4A and 4B show a plan view and a section view of a lower fixing unit of the first embodiment of this invention;
Figs. 5A and 5B show a plan view and a section view of a lower fixing unit of the second embodiment of this invention;
Figs. 6A and 6B show a plan view and a section view of an upper fixing unit of the first embodiment of this invention;
Figs. 7A and 7B show a lower plan view and a section view of an upper supporting unit of this invention;
Figs. 8A and 8B show a plan view and a section view of an lower supporting unit of this invention;
Fig. 9 shows a section view of a lower supporting unit of the second embodiment of this invention;
Fig. 10 shows a clamping apparatus according to the invention.

Hereinafter, the construction and operation of the invention are described with reference to the appending drawings.

This clamping apparatus is comprised of a fixing member for fixing the muscle and a pincette-shaped supporting member for applying force to the fixing member to enable the muscle to be fixed by the force applied fixing member. The fixing member includes an upper fixing unit 11 and a lower fixing unit 21, and the supporting member includes an upper supporting unit 31 and a lower supporting unit 41. The fixing member is made of bioabsorbable material such as polymer, etc. and the supporting member is made of metaline material.

Referring to Figs. 4A and 4B each showing a plan and a section of the lower fixing unit 21 according to the first embodiment of this invention, the lower fixing unit 21 is comprised of a body 22 having a plurality of holes 14 at regular intervals therebetween through which the sutures should be passed, and tapered projection 26 extended upward from the body 22. The body 22 is 0.08 to 0.15 mm thick in general. The upper part of the tapered projection 26 has an acute angle enough to pierce the muscle, and a groove 23 having a predetermined taper angle is formed at the inside of each projection 26. The projection 26 can be formed to be one unit with the body 22. However, it is also possible to attach the protrusion 26 produced by additional process to the body 22.

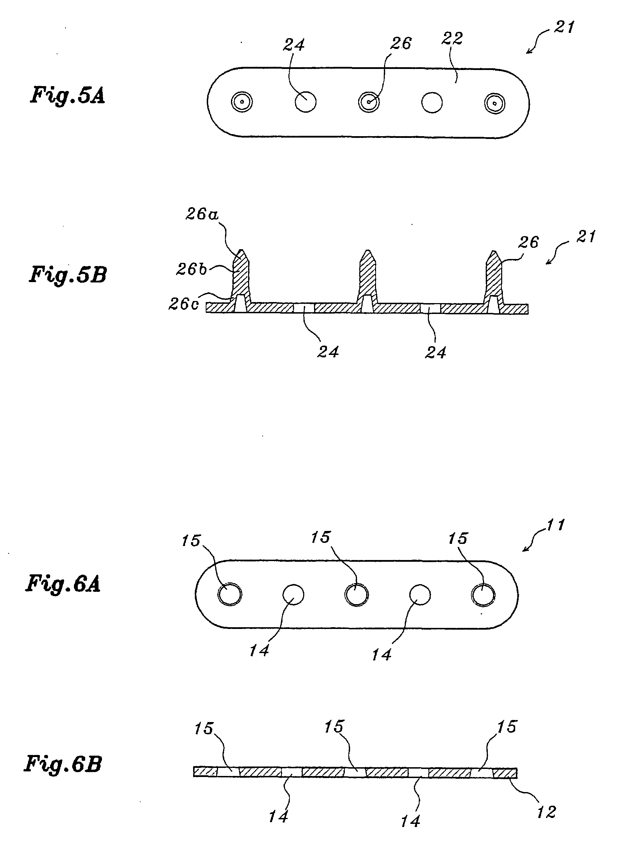

Figs. 5A and 5B show a plan and a section of a lower fixing unit 21 according to the second embodiment of this invention.

As shown in views, each projection 26 is comprised of three sections, a upper 26a, a middle 26b, a lower 26c, each having a different shape in such a manner that the upper portion 26a has an acute angle, the middle 26b has a cylinderlike shape having completely same diameter and the lower 26c is tapered.

Figs. 6A and 6B show a plan and a section of an upper fixing unit 11 according to the first embodiment of the this invention. In these views, the upper fixing unit 11 is comprised of a body 12 having a plurality of holes 14, 15. Noted that the holes 14, 15 refers as joint holes 14, 15 below. The body 12 is 0.08 to 0.15 mm thick in general, of which such thickness enables the joint holes 15 to be modified with ease without tear of the body 12 when the projection 26 of the lower fixing unit 21 are inserted to the holes of the upper fixing unit 11. In this embodiment, the base body 12 is shaped as a disposable bandage or the like.

Each through hole 24 of the upper fixing unit 11 is opposite to each through hole 24 of the lower fixing unit 21, and each joint hole 15 of the upper fixing unit 11 is opposite to each projection 26 of the lower fixing unit 21 to be associated with each other.

In this construction, it is desirable that the joint hole 15 where the projection 26 will be inserted is tapered to have a taper angle corresponding to the projection 26.

The diameter of the joint hole 15 can be varied depending on the thickness of the muscle. However it is designed to have a diameter identical with that of middle portion 26b of the tapered projection 26 in the first embodiment, while in the second embodiment to have a diameter identical with that of the cylindrical middle portion of the projection 26b.

The engagement of the projection 26 and the joint hole 15 is available if the taper angle of the tapered two part satisfies a self-locking condition to retain the projection 26 to the joint hole in assembled relation as a unit without getting free from the joint hole 15, the self-locking condition being defined by α ≤ ρ, where α is a taper angle and ρ is an angle of friction generated depending on the nature of material of which the projection 26 and the upper fixing unit 11 are made.

In this configuration, it is not necessary that the through hole 14 of the upper fixing unit 11 is designed to have a diameter equal to that of the through hole 24 of the lower fixing unit 21 and it is possible only if center points of two holes are formed on the same line to pass the suture therethrough.

On the other hand, the supporting member is to apply external force to the upper and lower fixing units 11, 21 to clamp the muscle between two fixing units 11, 21, and comprises the upper supporting unit 31, lower supporting unit 41 and a connection part, forming a shape like a pincette. The connection part is not shown in the appending drawings because it is not an essential element of this invention.

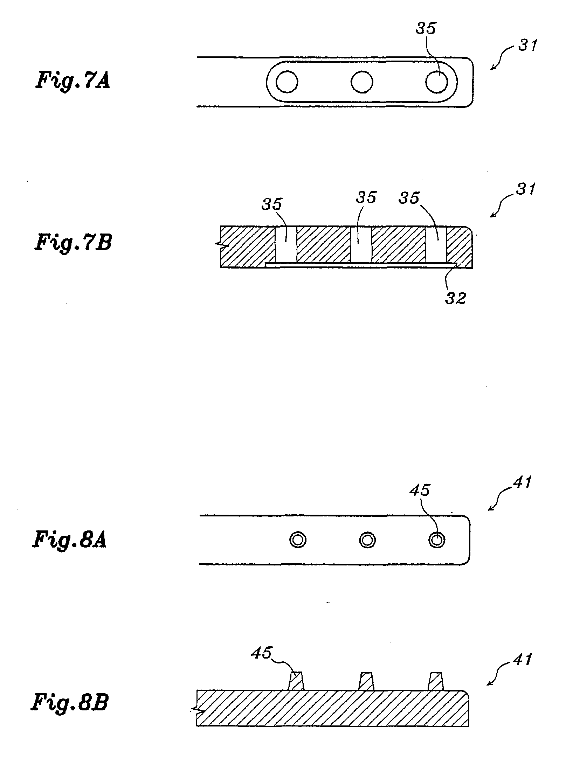

Next referring to Figs. 7A and 7B, the upper supporting unit 31 is provided with a recess 32 at the bottom where the upper fixing unit 11 is applied. The through hole 35 is further provided where the projection 26 of the lower fixing unit 21 is inserted. The recess 32 is designed to have a height equal to that of the upper fixing unit 11, and hence the upper fixing unit 11 and the upper supporting unit 21 in assembled relation as a unit have a flat bottom. The area of the recess 32 are slightly smaller than or equal to that of the upper fixing unit 11 just only to the extent that the upper fixing unit 11 dose not escape from the upper supporting unit 31 due to frictional force after the upper fixing unit 11 is inserted to the upper supporting unit 31.

The diameter of the through hole 35 is slightly larger than or equal to that of the lowermost portion of the tapered projection 26 so as to receive the entire protrusion 26 of the lower fixing unit 21.

Referring to Figs. 8A and 8B showing the lower supporting unit 41 of this invention, at the top surface of the lower supporting unit 41, there is a plurality of projections 45 each opposite to each groove 23 of the lower fixing unit 21. The respective projection 45 has a shape corresponding to the groove 23 to support the lower fixing unit 21. In this embodiment, the projection 45 is designed to be tapered while the diameter of the projection 45 varied upon the its height is slightly smaller than or equal to that of the groove 23 to assure the engagement of the lower fixing unit 21 and the lower supporting unit 41 by the frictional force.

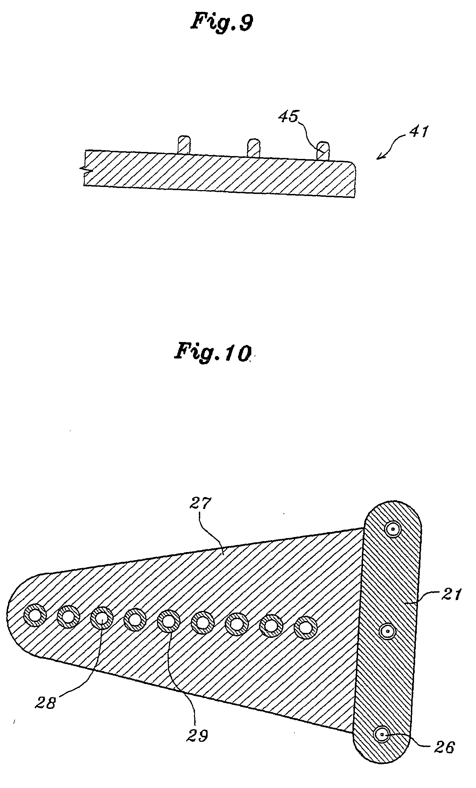

Fig. 9 shows another lower supporting unit part. The shape and height(depth) of the projection 45 are not important even if it can certainly secure the lower fixing unit 21 when inserted to the groove 23 of the lower fixing unit 21.

Fig. 10 shows a lower fixing unit 21 having membrane 27. On membrane 27, a plurality of through holes 28 for inserting the suture are made up in set distance, and the marginal spot of the said through hole forms the platform-elevated part 29, a raised place like a hump.

In case of operation by using membrane 27 only, the method of operation is simplified, but it is difficult to adjust exact length, so the suture can be used together. At this time, distance can be adjusted by using the through holes 28 for inserting the suture 1. That is, since the through holes 28 are made up in set distance, they can take a role of ruler, so that they can simply adjust distance by inserting the suture 1 into the through hole 28 in applicable distance.

Also, the platform-elevated part 29 is set up approximately equivalent to the thickness of the base part of the lower fixing unit 21, and as the suture 1 is put into the through hole, platform-elevated part prevents membrane 27 from tearing by the suture.

After the suture 1 is inserted into the applicable through hole 28, needless 51 remaining membrane 27 beyond the location of the through hole 28 is cut off, and the needle 51 is passed the center point of stump of muscle insertion, and tied.

Like this, this invention can eliminate side-effects like the piercing of the eyeball which can occur in case needle and suture are used, and manipulation is simple, and in case suture is used, it eliminates such a disadvantage that the width of the muscle is getting narrow according to tension of suture, keeping the primary level form of the muscle up as it is.

The clinical experiment results of operation to which this invention applies will be described as follows.

### [ THE CASE OF THE FIRST EXPERIMENT ]

For 13 colored tame rabbits of 2-3 kg, after the conjunctiva was exposed, the muscle was fixed to the superior and inferior rectus muscles by using the supporting unit and the fixing unit of the invention.

Subsequently, the adhesive portion of the superior rectus muscle is cut off by Westcott Scissors, and then in the rear of 3 mm, it is attached to the sclera by using BERIPLAST® (it is called recession), a tissue adhesive. Pulling tests have been carried out in 30 min., one week, two week, four week, eight week, respectively, gaining the results of measuring adhesive strength as follows :

| | 30 min | one week | two week | four week | eight week |
|---|---|---|---|---|---|
| adhesive strength | 110g | 210g | 300g | 480g | 650g |

Hence, adhesion proves firmly secured. As for the inferior rectus muscle, the results of examining adhesive width and tissue indicate that the width of the muscle is well maintained, and a slight degree of inflammation reaction occurs, but it declines slowly as one week, two weeks, four weeks, and eight weeks proceed on. Also, fibrosis increases, and in eight weeks, fibrin is developed between the muscle and the sclera, firmly attaching one and the other.

### [THE CASE OF THE SECOND EXPERIMENT]

For 13 colored tame rabbits of 2-3 kg, after fixing the muscle to the superior and inferior muscles by using the fixing unit and the supporting unit of the invention, and after taking it backward in the rear of 3 mm out of the muscle-adhesive spot by using the method of the hang-loose, as a result of observing the attached muscle in one week, two weeks, four weeks, and eight weeks, it becomes attached equivalent to the width of the primary muscle.

### [THE CASE OF THE THIRD EXPERIMENT]

With 20 healthy colored tame rabbits under general anesthesia, the conjunctiva of the ocular dexter (the right eye) is first incised, and the superior rectus muscle is detached as well. Then, the superior rectus muscle is tied up by 6-0 vinyl suture, and the portion in which the muscle is attached to the eyeball is cut out by Westcott Scissors, marking the sclera in the rear of 3 mm out of the muscle-adhesive spot by calipers while taking care of the sclera not to be pierced. Subsequently, after needle and suture are passed in to the half of the sclera in thickness and depth; suture is bound up with conventional recession technique; and the conjunctiva is resecured in its primary place.

In the ocular sinister (the left eye), the conjunctiva is equally cut off, the superior rectus muscle gets exposed, fixing the muscle by using the lower fixing unit and the upper fixing unit of the invention which have membrane, to cut off the portion attached to the eyeball by Westcott Scissors and thereby pass 6-0 vinyl suture, connecting it to end of membrane, and at the spot attached to the muscular sclera, the muscle is cut out, and out of which the center of the stump remaining is cut out by needle to be thereby fixed.

In 30 min, one week, two weeks, four weeks, and eight weeks after operation, the degree of conjunctiva hemorrhage and complications are examined. And after each of the superior rectus muscles of the right and left eyes(the right eye refers to the group using conventional type of hang-loose; the left eye to experimental group) lets exposed under general anesthesia, a pulling examination is performed.

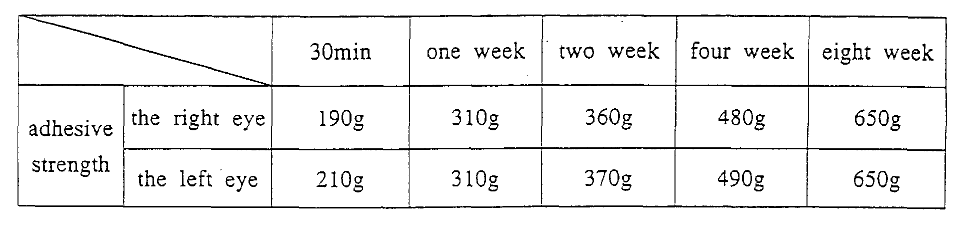

As a result, hemorrhage degree appears slight, and there appears no difference between both eyes.

## Claims

1. A muscle clamping apparatus comprising:
a first fixing means (21) having a plurality of projections (26) at the front thereof;
a second fixing means (11) having a plurality of joint holes (15) to which the respective projections of the first fixing means are inserted;
a first supporting means (41) for supporting the first fixing means; and
a second supporting means (31) having a plurality of through holes (35) for receiving the respective projections of the first fixing means, opposite to the first supporting means, for supporting the second fixing means,
**characterized in that**
a membrane (27) having a predetermined length and width area and including a plurality of suture inserting holes (28) at regular intervals therebetween is constructed as a unit body with the first fixing means (21).

2. A muscle clamping apparatus of Claim 1, wherein the first fixing means and the second fixing means further include suture passing holes (14, 24), the holes of both fixing means being formed at matching points to permit suture (1) to pass therethrough.

3. A muscle clamping apparatus of Claim 1, wherein each projection of the first fixing means has a tapered external surface and each joint hole of the second fixing means has a internal surface corresponding in angle of taper to the tapered external surface of the projection.

4. A muscle clamping apparatus of Claim 3, wherein each projection is comprised of;
a upper portion (26a) having a shaper taper angle;
a middle portion (26b) having a cylindrical shape; and
a lower portion (26c) having a shallow taper angle.

5. A muscle clamping apparatus of Claim 3 or 4, wherein the projections and the joint holes includes taper angle satisfying a self-locking condition.

6. A muscle clamping apparatus of Claim 3 or 4, wherein the first fixing means includes a groove (23) formed at the inward side of the projection and the first supporting means includes a projection (45) for inserting to the groove at the front.

7. A muscle clamping apparatus of Claim 6, wherein the second supporting means includes a recess (32) for securing the second fixing means therein.

8. A muscle clamping apparatus of Claim 3 or 4, wherein the first fixing means and the second fixing means are made of polymer.

## Patentansprüche

1. , Muskelklammervorrichtung, aufweisend:
ein erstes Fixiermittel (21), das eine Mehrzahl von Vorsprüngen (26) an seiner Vorderseite aufweist;
ein zweites Fixiermittel (11), das eine Mehrzahl von Verbindungslöchern (15) aufweist, in welche die jeweiligen Vorsprünge des ersten Fixiermittels eingesetzt sind;
ein erstes Stützmittel (41) zum Abstützen des erste Fixiermittels; und
ein zweites Stützmittel (31), das dem ersten Stützmittel gegenüberliegt, um das zweite Fixiermittel abzustützen, welches zweite Stützmittel eine Mehrzahl von Durchgangslöchern (35) zum Aufnehmen der jeweiligen Vorsprünge des ersten Fixiermittels aufweist,
**dadurch gekennzeichnet, daß**
eine Membran (27), die eine vorbestimmte Länge und Weitenfläche hat und eine Mehrzahl von Fadeneinsetzlöchern (28) in regelmäßigen Abständen dazwischen aufweist, als ein Einheitskörper mit dem ersten Fixiermittel (21) aufgebaut ist.

2. Muskelklammervorrichtung nach Anspruch 1, wobei das erste Fixiermittel und das zweite Fixiermittel ferner Fadendurchtrittslöcher (14, 24) aufweisen, wobei die Löcher beider Mittel an zusammenpassenden Stellen ausgebildet sind, um dem Faden (1) ein Passieren dahindurch zu ermöglichen.

3. Muskelklammervorrichtung nach Anspruch 1, wobei jeder Vorsprung des ersten Fixiermittels eine konische Außenfläche aufweist und jedes Verbindungsloch des zweiten Fixiermittels eine Innenfläche aufweist, die im Winkel des Konus der konischen Außenfläche des Vorsprungs entspricht.

4. Muskelklammervorrichtung nach Anspruch 3, wobei jeder Vorsprung aufweist:
einen oberen Abschnitt (26a) mit einem spitzen Konuswinkel;
einen mittleren Abschnitt (26b) mit einer zylindrischen Form; und
einen unteren Abschnitt (26c) mit einem stumpfen Konuswinkel.

5. Muskelklammervorrichtung nach Anspruch 3 oder 4, wobei die Vorsprünge und die Verbindungslöcher Konuswinkel aufweisen, die einem Selbstverriegelungszustand genügen.

6. Muskelklammervorrichtung nach Anspruch 3 oder 4, wobei das erste Fixiermittel eine Nut (23) aufweist, die an der Innenseite des Vorsprungs ausgebildet ist, und wobei das erste Stützmittel einen Vorsprung (45) zum Einsetzen in die Nut an der Vorderseite aufweist.

7. Muskelklammervorrichtung nach Anspruch 6, wobei das zweite Stützmittel eine Ausnehmung (32) zum Festlegen des zweiten Fixiermittels darin aufweist.

8. Muskelklammervorrichtung nach Anspruch 3 oder 4, wobei das erste Fixiermittel und das zweite Fixiermittel aus Polymer sind.

## Revendications

1. Dispositif de serrage de muscle comprenant :
un premier moyen de fixation (21) qui a une pluralité de saillies (26) dans sa partie avant,
un second moyen de fixation (11) qui a une pluralité de trous de jonction (15) dans lesquels sont insérées les saillies respectives du premier moyen de fixation,
un premier moyen de support (41) pour supporter le premier moyen de fixation, et
un second moyen de support (31) qui a une pluralité de trous continus (35) pour la réception des saillies respectives du premier moyen de fixation, opposé au premier moyen de support, pour le support du second moyen de support,
**caractérisé en ce que** :
une membrane (27) qui a une surface de longueur et de largeur prédéterminées et qui comprend une pluralité de trous d'insertion de suture (28) à intervalles réguliers entre ceux-ci est construite en tant que corps unitaire avec le premier moyen de fixation (21).

2. Dispositif de serrage de muscle selon la revendication 1, dans lequel le premier moyen de fixation et le second moyen de fixation comprennent en outre des trous de passage de suture (14, 24), les trous des deux moyens de fixation étant formés à des points coïncidents pour permettre à la suture (1) de passer à travers eux.

3. Dispositif de serrage de muscle selon la revendication 1, dans lequel chaque saillie du premier moyen de fixation a une surface externe conique et chaque trou de jonction du second moyen de fixation a une surface interne correspondant en angle de conicité à la surface externe conique de la saillie.

4. Dispositif de serrage de muscle selon la revendication 3, dans lequel chaque saillie comprend :
une partie supérieure (26a) ayant un angle de conicité plus aigu,
une partie centrale (26b) ayant une forme cylindrique, et
une partie inférieure (26c) ayant un angle de conicité peu profond.

5. Dispositif de serrage de muscle selon la revendication 3 ou 4, dans lequel les saillies et les trous de jonction comprennent un angle de conicité qui'satisfait à une condition d'auto-blocage.

6. Dispositif de serrage de muscle selon la revendication 3 ou 4, dans lequel le premier moyen de fixation comprend une rainure (23) formée du côté interne de la saillie et le premier moyen de support comprend une saillie (45) pour l'insertion dans la rainure à l'avant.

7. Dispositif de serrage de muscle selon la revendication 6, dans lequel le second moyen de support comprend un évidement (32) pour fixer le second moyen de fixation à l'intérieur de celui-ci.

8. Dispositif de serrage de muscle selon la revendication 3 ou 4, dans lequel le premier moyen de fixation et le second moyen de fixation sont constitués de polymère.
